Europäisches Patentamt

European Patent Office

Office européen des brevets

(11) Numéro de publication: **0 429 369 A1**

## DEMANDE DE BREVET EUROPEEN

(21) Numéro de dépôt: 90403307.3

(22) Date de dépôt: 22.11.90

(51) Int. Cl.⁵: **C12N 1/12, A01K 61/00**

(30) Priorité: 22.11.89 FR 8915314

(43) Date de publication de la demande:
29.05.91 Bulletin 91/22

(84) Etats contractants désignés:
BE DE ES GB IT NL

(71) Demandeur: **UNIVERSITE DE NANTES**
**1, Quai de Tourville**
**F-45035 Nantes Cédex 01(FR)**

(72) Inventeur: **Robert, Jean-Michel**
**29 Rue Castellano**
**F-44300 Nantes(FR)**

(74) Mandataire: **Dawidowicz, Armand Cabinet**
**Dawidowicz**
**18, Boulevard Pereire**
**F-75017 Paris(FR)**

(54) **Procédé de culture en masse de la diatomée Haslea ostrearia Simonsen.**

(57) L'invention concerne un procédé de culture en masse de la diatomée Haslea ostrearia Simonsen.

Selon l'invention, on prépare un milieu de culture constitué d'eau à une salinité et des concentrations en ammoniaque, éléments minéraux majeurs et oligo-éléments déterminés, on ramène le pH à au moins 7,30, on filtre, on choisit des algues issues de clones isolés et cultivés de manière spécifique, et on fait incuber les algues dans ce milieu de culture.

Application à la culture de l'algue de l'espèce Haslea ostrearia Simonsen.

EP 0 429 369 A1

## PROCÉDÉ DE CULTURE EN MASSE DE LA DIATOMÉE HASLEA OSTREARIA SIMONSEN.

L'invention concerne un procédé de culture en masse de la diatomée Haslea ostrearia Simonsen, également appelée "navicule bleue" ou algue bleue.

L'Haslea ostrearia est connue principalement pour son action de verdissement des huîtres du genre fines de claires". On peut penser qu'elle aurait d'autres applications dans le domaine des pigments des arômes, des produits alimentaires et cosmétiques, etc. mais sa présence aléatoire à l'état naturel n'a pas permis beaucoup de travaux dans cette direction.

Haslea ostrearia appartient à la classe des Diatomées, algues unicellulaires, dont déjà quelques espèces sont cultivées en aquaculture.

La culture et l'exploitation des algues unicellulaires marines a longtemps porté et porte encore sur un nombre restreint d'espèces dont on connaît bien la physiologie et dont la culture est relativement aisée : cellules de petite taille, mobiles, vivant en suspension dans le milieu, à paroi mince, faciles à traiter.

L'essor des biotechnologies marines et de l'aquaculture se trouve donc limité en l'état actuel à un certain nombre d'espèces qui se multiplient sans difficulté et dont on extrait un certain nombre de composés tels que pigments, protéines, vitamines, acides gras, etc. mais aucune de ces espèces ne permet le verdissement des huitres. On ressent donc vivement le besoin d'un procédé de culture d'un nouveau type qui permettrait la production en masse de cette algue en vue en particulier de son exploitation directe pour le verdissement des huîtres. L'industrie est donc à la recherche d'une telle production en masse de manière à satisfaire des demandes de plus en plus pressantes d'algues marines.

L'invention a donc pour but de fournir un procédé de culture de l'algue bleue dans des volumes de milieu de plusieurs mètres cubes pour mieux répondre à une exploitation de type industriel.

Elle a aussi pour but d'utiliser le milieu après culture de l'algue pour le verdissement des huîtres : celui-ci est en effet teinté en bleu vert par le pigment libéré par l'algue, la marennine.

A cet effet, le procédé selon l'invention est caractérisé en ce que
- on prépare un milieu de culture constitué d'eau salée ayant une salinité comprise entre 25 et 34 °/oo et une concentration en ammoniaque inférieure à 110 $\mu$ M/dm$^3$, des concentrations en éléments minéraux majeurs telles que :
azote nitrique : entre 60 et 150 $\mu$ M/dm$^3$ phosphore : entre 3 et 6 $\mu$ M/dm$^3$ silicium : entre 60 et 200 $\mu$ M/dm$^3$ des concentrations en oligo-éléments métalliques telles que :
cuivre : entre 2.10$^{-8}$ et 6.10$^{-8}$ M/dm$^3$
fer : entre 5.10$^{-8}$ et 20.10$^{-6}$ M/dm$^3$
zinc : entre 6.10$^{-8}$ et 12.10$^{-8}$ M/dm$^3$
nickel : entre 1.10$^{-8}$ et 2.10$^{-8}$ M/dm$^3$,
- on ramène le pH du milieu à une valeur supérieure ou égale à 7,30,
- on filtre le milieu à une maille de 0,45 $\mu$m,
- on choisit des algues issues de clones isolés et mis en culture en laboratoire dans des récipients en verre à une température de l'ordre de 18° C, avec un éclairement de l'ordre de 3000 lux à un rythme de 14 heures de lumière suivies de 10 heures d'obscurité, pendant 6 jours,
- on fait incuber les algues sous serre dans des bacs circulaires de 300 à 500 dm$^3$ de manière à maintenir la température entre 12° C et 25° C, en éclairement naturel, sans agitation, en milieu calme, les bacs étant recouverts de toile à bluter, pendant 8 jours.

Dans ces conditions, on atteint dans les bacs de 300 dm$^3$ une biomasse de 60 à 120.10$^6$ cellules/dm$^3$, avec une couleur bleu-vert caractéristique de la production par l'algue du pigment bleu surnuméraire. Cette biomasse représente une masse fraîche d'algues de 20g pour 100 dm$^3$ de culture, avec une teneur en eau comprise entre 70 et 90 % après concentration par centrifugation avec cylindre clarificateur.

Selon une forme de mise en oeuvre du procédé selon l'invention, le milieu de culture est constitué d'eau de mer souterraine additionnée de nitrates pour obtenir la concentration en azote voulue.

De préférence, la concentration en ammoniaque du milieu de culture est inférieure ou égale à 80 $\mu$ M/dm$^3$. Avantageusement, la salinité du milieu de culture est comprise entre 28 et 32 °/oo.

Selon une forme de mise en oeuvre, les concentrations en éléments minéraux essentiels sont :
azote nitrique : environ 100 $\mu$ M/dm)$^3$
phosphore : environ 4 $\mu$ M/dm$^3$
silicium : environ 100 $\mu$ M/dm$^3$.

Avantageusement, les concentrations en oligo-éléments métalliques sont :
cuivre : environ 4 .10$^{-8}$ M/dm$^3$
fer : environ 12 .10$^{-6}$ M/dm$^3$
zinc : environ 8 .10$^{-6}$ M/dm$^3$
nickel : environ 1,5.10$^{-6}$ M/dm$^3$

La culture de la diatomée Haslea ostrearia Simonsen ainsi effectuée conformément à l'invention se caractérise par la production du pigment bleu qui diffuse en partie dans le milieu de culture, ce qui lui donne cette couleur bleu-vert caractéristique. La concentration en pigment bleu dans le milieu de culture, après concentration des algues, pour les cultures de 300 dm³, est de l'ordre de 130 mg/dm³. L'eau verte ainsi produite peut être utilisée après filtration, pour le verdissement d'huîtres, processus qui s'effectue naturellement dans l'affinage des huîtres "fines de claires". Cette eau conduit au verdissement des huitres en 12 heures, pour une proportion d'environ 1 huître/dm³ d'eau. L'eau peut ensuite servir au verdissement d'un autre lot d'huîtres.

Le procédé de culture en masse selon l'invention permet d'obtenir simultanément, en masse, de l'eau de mer pour le verdissement des huîtres, et des algues bleues qui peuvent être conservées par congélation ou lyophilisation ou qui peuvent être traitées, en particulier pour en extraire le pigment bleu.

Pour le verdissement des huîtres, on peut opérer en bacs du type "race-way" permettant un verdissement accéléré. On peut également effectuer un écoulement constant dans des claires ostréicoles attenantes à la structure de production des algues, pour le verdissement d'huîtres du type "fine de claire" ou du type "spéciale"

## Revendications

1. Procédé de culture en masse de la diatomée Haslea ostrearia Simonsen,
caractérisé en ce que
- on prépare un milieu de culture constitué d'eau salée ayant une salinité comprise entre 25 et 34 °/oo et une concentration en ammoniaque inférieure à 110 $\mu$ M/dm³, des concentrations en éléments minéraux majeurs telles que :
azote nitrique : entre 60 et 150 $\mu$ M/dm³
phosphore : entre 3 et 6 $\mu$ M/dm³
silicium : entre 60 et 200 $\mu$ M/dm³
des concentrations en oligo-éléments métalliques telles que :
cuivre : entre $2.10^{-8}$ et $6.10^{-8}$ M/dm³
fer : entre $5.10^{-8}$ et $20.10^{-6}$ M/dm³
zinc : entre $6.10^{-8}$ et $12.10^{-8}$ M/dm³
nickel : entre $1.10^{-8}$ et $2.10^{-8}$ M/dm³,
- on ramène le pH du milieu à une valeur supérieure ou égale à 7,30,
- on filtre le milieu à une maille de 0,45 $\mu$m,
- on choisit des algues issues de clones isolés et mis en culture en laboratoire dans des récipients en verre à une température de l'ordre de 18° C, avec un éclairement de l'ordre de 3000 lux à un rythme de 14 heures de lumière suivies de 10 heures d'obscurité, pendant 6 jours,
- on fait incuber les algues sous serre dans des bacs circulaires de 300 à 500 dm³ de manière à maintenir la température entre 12° C et 25° C, en éclairement naturel, sans agitation, en milieu calme, les bacs étant recouverts de toile à bluter, pendant 8 jours.

2. Procédé selon la revendication 1,
caractérisé en ce que le milieu de culture est constitué d'eau de mer souterraine additionnée de nitrates pour obtenir la concentration en azote voulue.

3. Procédé selon l'une des revendications 1 et 2,
caractérisé en ce que la concentration en ammoniaque du milieu de culture est inférieure ou égale à 80 $\mu$ M/dm³.

4. Procédé selon l'une des revendications 1 à 3,
caractérisé en ce que la salinité du milieu de culture est comprise entre 28 et 32 °/oo.

5. Procédé selon l'une des revendications 1 à 4,
caractérisé en ce que les concentrations en éléments minéraux essentiels sont :
azote nitrique : environ 100 $\mu$ M/dm³
phosphore : environ 4 $\mu$ M/dm³
silicium : environ 100 $\mu$ M/dm³.

6. Procédé selon l'une des revendications 1 à 5,
caractérisé en ce que les concentrations en oligo-éléments métalliques sont :
cuivre : environ $4 .10^{-8}$ M/dm³
fer : environ $12 .10^{-6}$ M/dm³
zinc : environ $8 .10^{-8}$ M/dm³
nickel : environ $1,5.10^{-8}$ M/dm³.

7. Procédé selon l'une des revendications 1 à 6,
caractérisé en ce qu'on sépare les algues produites d'avec l'eau enrichie de pigments bleus.

8. Procédé de verdissement des huîtres,
caractérisé en ce qu'on place des huîtres dans l'eau obtenue par le procédé selon la revendication 7, en proportion d'environ une huître par dm³ d'eau, et on laisse les huîtres pendant environ 12 heures dans ladite eau.

9. Procédé selon la revendication 8,
caractérisé en ce que le verdissement est effectué dans des bacs du type "race-way".

10. Procédé selon la revendication 8,
caractérisé en ce qu'on effectue un écoulement constant de ladite eau dans des claires ostréicoles attenantes à la structure de culture en masse des algues.

11. Algue de l'espèce haslea ostrearia Simonsen,
caractérisée en ce qu'elle est obtenue par le procédé selon l'une des revendications 1 à 10.

12. Application de l'algue selon la revendication 11 au verdissement des huitres.

Office européen
des brevets

RAPPORT DE RECHERCHE
EUROPEENNE

Numéro de la demande

**EP 90 40 3307**

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | Comptes Rendus des Séances de l'Académie des Sciences.Série D.t271. no. 25, 21 décembre 1970, PARIS pages 2389 - 2391; NEUVILLE D. et al: "Observations concernant la production de pigment bleu par la diatomée N.ostrearia Bory maintenue en culture uni-algale"<br>* le document en entier *<br>— — — | | C<br>12 N 1/12<br>A 01 K 61/00 |
| A | Comptes Rendus des Séances de l'Académie des Sciences.Série D.t.287. no. 16, 18 décembre 1978, PARIS. pages 1413 - 1415; BAGES M. et al: "Biomasse et fertilité des claires à Huîtres de la baie de Bourgneuf(Vendée)"<br>* le document en entier *<br>— — — | | |
| A | Oceanologica Acta vol. 4, no. 1, 1981, MONTREUIL.FR. pages 13 - 21; MAESTRINI S.Y. et al: "Rendements d'utilisatipn des sels nutritifs et variations de l'etat des cellules de 3 diatomées de claires à huîtres de Vendée"<br>* le document en entier *<br>— — — | | |
| A | Comptes Rendus des Séances de l'Academie des Sciences.série D.t. 274. 05 avril 1972, PARIS. pages 2030 - 2033; NEUVILLE D. et al: "Production du pigment bleu par la Diatomée N.ostrearia Bory maintenue en culture uni-algale sur un milieu carencé en azote nitrique"<br>* le document en entier *<br>— — — — — | | DOMAINES TECHNIQUES RECHERCHES (Int. Cl.5)<br><br>C 12 N<br>A 01 G |

Le présent rapport de recherche a été établi pour toutes les revendications

| Lieu de la recherche | Date d'achèvement de la recherche | Examinateur |
|---|---|---|
| La Haye | 20 février 91 | LE CORNEC N.D.R. |